# EUROPÄISCHE PATENTSCHRIFT

(11) **EP 1 309 335 B1**
(45) Veröffentlichungstag und Bekanntmachung des Hinweises auf die Patenterteilung: **10.03.2004**
(21) Anmeldenummer: 01956251.1
(22) Anmeldetag: 02.08.2001
(51) Int. Cl.: A61K 35/78, A61P 25/00

(54) **VERWENDUNG VON EXTRAKTEN AUS GINKGO BILOBA-BLÄTTERN ZUR TRAUMPROVOKATION**
USE OF EXTRACTS FROM GINKGO BILOBA LEAVES FOR INDUCING DREAMS
UTILISATION D'EXTRAITS DE FEUILLES DE GINKGO BILOBA POUR SUSCITER DES REVES

(30) Priorität: 17.08.2000 CH 160400
(43) Veröffentlichungstag der Anmeldung: 14.05.2003
(73) Patentinhaber: MEDICHEMIE AG, CH-4107 Ettingen/Basel (CH)
(72) Erfinder: WIDAUER, Josef, Olav, CH-4123 Allschwil (CH); PÖLDINGER, Walter, CH-2344 Maria Enzersdorf (CH)
(74) Vertreter: Lauer, Joachim
(86) Internationale Anmeldenummer: PCT/CH2001/000473
(87) Internationale Veröffentlichungsnummer: WO 2002/013841

(56) Entgegenhaltungen:
- EP-A- 0 143 977
- EP-A- 0 436 129
- DATABASE BIOSIS [Online] BIOSCIENCES INFORMATION SERVICE, PHILADELPHIA, PA, US; Mai 1999 (1999-05) CURTIS-PRIOR PETER ET AL: "Therapeutic value of Ginkgo biloba in reducing symptoms of decline in mental function." Database accession no. PREV199900341291 XP002155527 & JOURNAL OF PHARMACY AND PHARMACOLOGY, Bd. 51, Nr. 5, Mai 1999 (1999-05), Seiten 535-541, ISSN: 0022-3573

## Beschreibung

### TECHNISCHES GEBIET

Die vorliegende Erfindung bezieht sich auf das Gebiet der psychoanalytischen Therapie. Sie betrifft insbesondere ein Mittel zur Steigerung der Traumaktivität im Sinne einer Traumprovokation sowie zu einer Verbesserung der Erinnerungsfähigkeit an die gehabten Träume.

### STAND DER TECHNIK

Extrakte der Blätter von Gingko biloba, einem aus China stammenden Zierbaum, werden von alters her für diverse medizinische Anwendungen eingesetzt. Neben den alternativmedizinischen und homöopatischen Verwendungen sind im klassisch medizinischen Bereich unter anderem folgende Indikationen von Gingko biloba Extrakten bekannt: Durchblutungsförderung durch Verminderung der Plasmaviskosität und der Thrombocytenaggregation sowie Erhöhung der Kapillarpermeabilität, Förderung der Kompensation von Gleichgewichtsstörungen, Förderung der Durchblutung, insbesondere der Mikrozirkulation, Steigerung der Hypoxietoleranz, insbesondere des Himgewebes, sowie Verminderung des Retinaödems von Netzhautläsionen, und Steigerung der Gedächtnisleistung und des Lernvermögens.

Die medikamentöse Verwendung zur Steigerung von Traumaktivitäten und belastender Erlebniskomplexe an Patienten wurde in früheren Jahren mittels der Psycholyse durchgeführt. In der Psycholyse wurden dem Patienten niedrige Dosen von Halluzinogenen, beispielsweise Lysergsäurediethylamid (LSD), Psylocibin oder Mescalin kontrolliert verabreicht. Die bei den Patienten auftretenden lebhaften Illusionsverkennungen, wurden anschliessend wie Träume mittels freier Assoziation mit dem Psychotherapeuten analytisch verarbeitet.

Auf Grund der hohen Nebenwirkungen und den grossen Missbräuchen der verwendeten Halluzinogene, finden diese keine therapeutische Anwendung mehr. Von daher besteht das Bedürfnis, ein wirksames Mittel zur Verfügung zu stellen, das eine verstärkte Traumaktivität hervorruft und welches möglichst geringe Nebenwirkungen hat.

### DARSTELLUNG DER ERFINDUNG

Es ist bekannt, dass die Verabreichung von Trockenextrakten aus Blättern von Ginkgo biloba insbesondere eine signifikante Hirnleistungssteigerung hervorruft.

Überraschenderweise zeigten Patienten, welche eine den üblichen Bereich überhöhte Dosis des Ginkgo biloba Extraktes zu sich nahmen, eine auffallende lebhafte, sehr intensive Traumaktivität bzw. eine Traumerlebnissteigerung. Des weiteren war auffallend, dass die Träume den Patienten sehr gut in Erinnerung blieben.

Diese Ergebnisse konnten in Studien mit mehreren männlichen Probanden verifiziert werden, wobei die tägliche Dosis des Ginkgo biloba Extraktes (Symfona N, 120mg) zwischen 360-480 mg lag.

Gegenstand der Erfindung ist die Verwendung eines Extrakts aus Ginkgo biloba-Blättern zur Herstellung eines Mittels zur Traumprovokation.

Insbesondere erweist sich die obige Verwendung eines Extrakts aus Ginkgo biloba-Blättern als geeignet zur therapeutischen Anwendung im Bereich der psychoanalytischen Behandlung. Die gemeinsame Verarbeitung von Traumerlebnissen mit dem Therapeuten spielt dabei eine wichtige Rolle. Demnach ist der Ginkgo biloba Extrakt nach der Erfindung besonders gut geeignet für Patienten, die eine ungenügende Traumaktivität aufweisen.

Ein zusätzlicher Nutzen besteht beim Einsatz des erfindungsgemässen Ginkgo biloba durch die Verbesserung der Erinnerungsfähigkeit an die gehabten Träume.

Für die obigen Verwendungen wird vorzugsweise ein Extrakt unter Zuhilfenahme eines Aceton/Wasser Gemisches hergestellt, wie es in der Aufbereitungsmonographie für einen Trockenextrakt aus Ginkgo biloba-Blättem für den humanmedizinischen Bereich des deutschen Bundesgesundheitsamtes (Bundesanzeiger Nr. 133, 19.07.1994) aufgezeigt wird.

### WEGE ZUR AUSFÜHRUNG DER ERFINDUNG

Beispielhaft mögliche Formulierungen der verabreichten Ginkgo biloba-Präparate (Kapseln, Filmtabletten, Tropfen und Sirup) sind folgende (alle unter Verwendung eines Extraktes wie es in der Aufbereitungsmonographie für ein Trockenextrakt aus Ginkgo biloba-Blättern für den humanmedizinischen Bereich des deutschen Bundesgesundheitsamtes, Bundesanzeiger Nr. 133, 19.07.1994, vorgeschlagen wird):

### Beispiel 1: Kapseln

15 kg Ginkgo biloba Trockenextrakt werden mit Milchzucker, hochdispersem Siliciumdioxid und Magnesiumstearat gemischt und in 125 000 Hartgelatinekapseln mit einem Gehalt von je 120 mg Ginkgo biloba Trockenextrakt abgefüllt.

### Beispiel 2: Filmtabletten

15 kg Ginkgo biloba Trockenextrakt werden mit Maisstärke, hochdispersem Siliciumdioxid, Milchzucker, Zellulose, Dicalciumphosphat, Magnesiumstearat und Talk gemischt und zu Tabletten mit einem Gehalt von je 120 mg Ginkgo biloba Trockenextrakt gepresst. Diese Tabletten werden mit einem Film überzogen, der aus Polymeren wie Zellulosederivaten. Polyethylenglykol. Talk und Farbstoffen für den Lackfilm besteht.

### Beispiel 3: Tropfen

9 kg Ginkgo biloba Trockenextrakt werden in Propylenglykol gelöst. Diese Lösung wird mit einer wässrigen Lösung vermischt, die verschiedene Süssstoffe, Aromatika, Geschmackskorrigentien und Puffersalze enthält.

### Beispiel 4: Sirup

0,9 kg Ginkgo biloba Trockenextrakt werden in Propylenglykol gelöst und mit einer wässrigen Lösung vermischt, die aus Glycerol, verschiedenen Süssstoffen, Aromenstoffen und Puffersalzen besteht.

In den vorstehenden Beispielen enthalten die Kapseln und die Filmtabletten jeweils 120 mg Ginkgo biloba Trockenextrakt, der auf mindestens 25 % Flavonglykoside und mindestens 6 % Terpenlaktone standardisiert ist, das heisst auf mindestens 30 mg Flavonglykoside und 7,2 mg Terpenlaktone je Darreichungsform. Die Tropfen und der Sirup enthalten 60 mg bzw. 6 mg des Trockenextrakts pro ml Lösung. Anwendung finden beispielsweise die von der Anmelderin unter dem Namen Symfona-N in der Schweiz vertriebenen Produkte, welche Ginkgo biloba Extrakt enthalten.

Eine Verabreichung von dreimal täglich je einer Kapsel bzw. Filmtablette, von 2 ml Tropfen oder 20 ml Sirup entspricht einer mittleren Tagesdosis von 360 mg Trokkenextrakt. Für die hier beschriebenen Anwendungen sind mittlere Tagesdosen in einem Bereich, welcher 240 - 480mg Trockenextrakt entspricht, sinnvoll einsetzbar.

## Patentansprüche

1. Verwendung von Extrakten aus Ginkgo-biloba-Blättern zur Herstellung eines Arzneimittels zur Steigerung der Traumaktivität eines Patienten im Rahmen einer Traumerlebnisse verarbeitenden psychoanalytischen Theraple.

2. Verwendung von Extrakten aus Ginkgo-biloba-Blättern zur Herstellung eines Arzneimittels zur Verbesserung der Erinnerungsfähigkelt eines Patienten an gehabte Träume im Rahmen einer Traumerlebnisse verarbeitenden psychoanalytischen Therapie.

3. Verwendung eines Extraktes nach den Ansprüchen 1 oder 2, **dadurch gekennzeichnet, dass** es sich um einen Trockenextrakt aus Ginkgo biloba-Blättern, extrahiert mit Aceton-Wasser, handelt, welcher der Aufbereitungsmonographie des deutschen Bundesgesundheitsamtes für den humanmedinischen Bereich entspricht

## Claims

1. A use of extracts of Ginkgo biloba leaves for manufacturing a medicinal product for increasing the dream activity of a patient as part of a psychoanalytic therapy which assimilates a dream experience.

2. The use of extracts of Ginkgo biloba leaves for manufacturing a medicinal product for improving the memory capability of a patient for dreams they have had as part of a psychoanalytic therapy which assimilates a dream experience.

3. The use of an extract according to claims 1 or 2, **characterised in that** this is a dry extract of Ginkgo biloba leaves extracted with acetone-water, which conforms to the preparation monograph of the German Federal Health Office for the area of human medicine.

## Revendications

1. Utilisation d'extraits de feuilles de ginkgo biloba pour la fabrication d'un médicament destiné à augmenter l'activité onirique d'un patient dans le cadre d'une thérapie psychanalytique, traitant les évènements oniriques.

2. Utilisation d'extraits de feuilles de ginkgo biloba, pour la fabrication d'un médicament destiné à améliorer la capacité de mémoire d'un patient concernant les rêves vécus dans le cadre d'une thérapie psychanalytique, traitant les évènements oniriques.

3. Utilisation d'un extrait selon l'une quelconque des revendications 1 ou 2, **caractérisée en ce qu'**il s'agit d'un extrait sec de feuilles de ginkgo biloba, extrait à l'aide d'eau et d'acétone et correspondant à la monographie de préparation de l'Office Fédéral Allemand d'Hygiène publique pour le secteur de la médecine humaine.
